Europäisches Patentamt

European Patent Office

Office européen des brevets

(19)

(11) Numéro de publication: **0 363 491 B1**

(12) **FASCICULE DE BREVET EUROPEEN**

(45) Date de publication de fascicule du brevet: **02.02.94** (51) Int. Cl.5: **A61K 35/74**

(21) Numéro de dépôt: **89901921.0**

(22) Date de dépôt: **04.11.88**

(86) Numéro de dépôt internationale :
**PCT/SU88/00223**

(87) Numéro de publication internationale :
**WO 89/09607 (19.10.89 89/25)**

(54) **PREPARATION BACTERIENNE POUR PROPHYLAXIE ET TRAITEMENT DE PROCESSUS INFLAMMATOIRES ET D'AFFECTIONS ALLERGIOUES.**

(30) Priorité: **15.04.88 SU 4426998**

(43) Date de publication de la demande:
**18.04.90 Bulletin 90/16**

(45) Mention de la délivrance du brevet:
**02.02.94 Bulletin 94/05**

(84) Etats contractants désignés:
**AT BE CH DE FR GB IT LI LU NL SE**

(56) Documents cités:
**EP-A- 0 228 861**
**GB-B- 1 434 582**
**GB-B- 1 486 508**
**GB-B- 1 585 863**

**Antibiotiki i meditsinskaya biotekhnologia, vol. XXXI, No. 9, Septembre 1986 (Meditsina, Moscou), L.G. Zaitseva et al., pp. 691-694**

(73) Titulaire: **NIKITENKO, VYACHESLAV IVANO-VICH**
**ulitsa Volodarskogo, 27, kv. 53**
**Orenburg(RU)**

(72) Inventeur: **NIKITENKO, Vyacheslav Ivanovich**
**ul. Volodarskogo, 27-53**
**Orenburg, 460000(SU)**
Inventeur: **NIKITENKO, Ivan Kirillovich**
**ul. Cheljuskintsev, 17d-26**
**Orenburg, 460014(SU)**
Inventeur: **NIKITENKO, Ljubov Ivanovna**
**ul. Volodarskogo, 27-53**
**Orenburg, 460000(SU)**
Inventeur: **PAU, Sergei Mikhailovich**
**ul. Chkalova, 36-113**
**Orenburg, 460001(SU)**

EP 0 363 491 B1

Veterinaria, No. 7, juillet 1987, (VO "Agropromizdat, Moscou), V.M. Karpov, p. 45 indiqué dans la description

Nauka i zhizn, No. 9, septembre 1988, (Pravda, Moscou), p. 120, 1-ère colonne

(74) Mandataire: **Durand, Yves Armand Louis et al**
**CABINET WEINSTEIN**
**20, Avenue de Friedland**
**F-75008 Paris (FR)**

## Description

La présente invention concerne la médecine et la médecine vétérinaire, et plus précisément, une nouvelle préparation bactérienne pour la prophylaxie et le traitement des processus inflammatoires et des affections allergiques.

On connaît actuellement un nombre de préparations bactériennes curatives et prophylactiques vivantes telles que la colibactérine obtenue à base de la souche Escherichia coli M 17, la bifidumbactérine à base de la souche B. bifidum, la lactobactérine à base de la culture Lactobacillus plantarum 8 PA-3, la bactérine SL à base des souches du genre Bacillus, la bactisubtile etc. Toutes les préparations indiquées sont destinées à la prophylaxie et au traitement des infections bactériennes du tractus gastro-intestinal et de la dysbactériose de l'homme et des animaux (aide-mémoire de l'emploi de préparations bactériennes et virales, sous la rédaciton de S.G. Dzagurov et de F.F. Resepov, 1975 (Moscou), Ed. "Meditsina" ; V.M. Karpov, Préparation "Bactérine SL", Médecine vétérinaire, 1987 (Moscou), 7, p. 45).

Aussi, connaît-on certaines tentatives de l'utilisation locale des bactéries vivantes pour la prophylaxie et le traitement des processus inflammatoires. Les bactéries sont introduites à l'endroit de la lésion. (G.I. Khanine et al "Utilisation des bifido-et lactobactéries pour la correction de la microécologie des voies génitales et les particularités technologiques expérimentales de l'élaboration à leur base des préparations curatives", Bifidobactéries et leur utilisation dans la clinique, l'industrie médicale et l'agriculture, 1986 (Moscou) pp. 151-156). Les préparations bactériennes indiquées (colibactérine, bifidumbactérine, bactisubtile) ont une faible activité antagoniste vis-a-vis des agents pathogènes et un faible pouvoir de production des enzymes protéolytiques. En outre, en cas d'utilisation locale pour la prophylaxie et le traitement de la suppuration des plaies, les préparations indiquées n'influent pas sur les agents pathogènes dans les tissus qui, comme on le sait, jouent un rôle principal dans le développement du processus pathologique, ainsi que sur des couches profondes de la nécrose. Cela réduit brusquement leur effet curatif et prophylactique.

On connaît également, dans le brevet britannique N° 1 585 863 des préparations à usage pharmaceutique à base d'une ou plusieurs souches de lactobacillus pour la prévention d'infection ou d'inflammation, ou pour enrayer des inflammations ou des maladies infectieuses telles que l'appendicite, la gastrite, l'entérite, la gingivite et les hémorroïdes.

La demande de brevet européen N° 228 861 indique l'utilisation de nouveaux produits cellulaires bactériens à base de micoorganismes du genre Streptococcus, Bifidobactérium et Lactobacillus et ayant des pouvoirs anticancérigènes.

La préparation bactérienne selon l'invention pour la prophylaxie et le traitement des processus inflammatoires et des affections allergiques, est une préparation nouvelle qui n'est pas décrite dans la littérature.

On s'est proposé de créer une nouvelle préparation bactérienne possédant une haute activité, un large spectre d'action et n'ayant pas de réactions secondaires.

La solution consiste en ce que la préparation bactérienne selon l'invention, pour la prophylaxie et le traitement des processus inflammatoires et des affections allergiques présentent des cellules d'au moins un micro-organisme de bactéries choisi parmi les souches Bacillus subtilis 534, Bacillus sp. 538, Bacillus pulvifaciens 535 possédant un pouvoir de pénétrer du tractus gastro-intestinal au foyer de la lésion, de conserver sa viabilité et de produire des antibiotiques, des enzymes protéolytiques, des modulateurs d'immunité et contient de 5 à $1.10^9$ cellules vivantes de micro-organismes par mg de préparation.

La souche Bacillus subtilis 534 utilisée dans la préparation bactérienne, a été déposée le 28.03.88 auprès de la Collection pansoviétique des micro-organismes de l'Institut biokhimii i fiziologii mikroorganizmov de l'Académie des Sciences de l'URSS et enregistrée sous le numéro B-1666 II.

La souche Bacillus sp. 538 utilisée dans la présente invention a été déposée le 20.06.88 auprès de la Collection pansoviétique des micro-organismes du Vsesojuzny nauchno-issledovatelsky institut genetiki i selektsii promyshlennykh mikroorganizmov et enregistrée sous le numéro BK II M-4401.

La souche Bacillus pulvifaciens 535 utilisée dans la préparation bactérienne selon l'invention a été déposée le 14.04.88 auprès de la Collection pansoviétique des micro-organismes industriels du Vsesojuzny nauchno-issledovatelsky institut genetiki i selektsii promyshlennykh mikroorganizmov et enregistrée sous le numéro BK II M-4348.

La présente invention concerne également l'utilisation de la souche Escherichia Coli M17 pour l'obtention d'une préparation bactérienne médicamenteuse selon la présente invention destinée à traiter des processus purulents dans les tissus musculaires des extrémités.

La préparation selon l'invention peut remplacer les antibiotiques, les enzymes protéolytiques et, dans le cas de certaines affections, les moyens désensibilisants. La préparation est administrée per os et se concentre au foyer pathologique. Dans les tissus du milieu interne de l'organisme, la préparation inhibe la croissance des bactéries gram-positives et gram-négatives, des microbactéries, favorise la purification à partir des tissus nécrosés, arrête les réactions allergiques.

La préparation selon l'invention est obtenue par une technologie simple.

La préparation selon l'invention peut être conservée à une température allant jusqu'à 100°C, durant 10 ans et au-delà, tandis que le délai d'aptitude des préparations bactériennes connues, des antibiotiques, des enzymes, ne dépasse pas deux ans dans les conditions de régime de température plus sévères. La préparation selon l'invention, ne donne pratiquement pas de réactions secondaires, étant donné qu'elle est réalisée à base d'un phénomène largement répandu mais auparavant inconnu dans la nature, notamment le phénomène de protection bactérienne naturelle. La préparation revendiquée a un large spectre d'action et, en outre, comme les produits connus, est efficace en cas de dysbactérioses, de perturbations de digestion.

La préparation selon l'invention, est préparée à partir de micro-organismes capables de pénétrer du foyer d'introduction dans les tissus pathologiquement modifiés de l'organisme chez l'homme, les animaux et les oiseaux, y conserver sa viabilité, de dégager des substances biologiquement actives inhibant le processus pathologique ou empêchant son développement.

On isole les bactéries vivantes à partir de tissus de l'organisme chez l'homme, les animaux et les oiseaux.

On choisit parmi les bactéries celles qui peuvent pénétrer du tractus gastro-intestinal au foyer pathologique ou aux organes et aux tissus sains strictement déterminés. Les souches obtenues sont examinées sur la pathogénicité, la virulence, la toxicité aiguë et chronique, l'action allergène, tératogène et oncogène. En cas d'absence d'actions secondaires indésirables, les souches des microorganismes peuvent être utilisées pour la production de la préparation bactérienne.

Outre cela, on détermine le pouvoir des souches de conserver leur viabilité dans les tissus et de dégager les substances biologiquement actives, antibiotiques, enzymes protéolytiques, modulateurs d'immunité. On obtient à base de souches choisies, une préparation bactérienne par culture sur des milieux nutritifs liquide ou solide. Ultérieurement, on l'utilise à l'état natif ou à l'état desséché avec les accumulateurs ou sans ceux-ci. La préparation peut contenir une ou plusieurs souches de bactéries à raison de 5 à $1.10^9$ cellules vivantes de micro-organismes par mg. Pour la production de la préparation, on utilise de préférence les nouvelles souches suivantes : souche Bacillus subtilis 534, souche Bacillus s.p. 538, souche Bacillus pulvifaciens 535. Il est avantageux d'utiliser également des souches connues telles que Escherichia coli M17. La nouvelle souche Bacillus subtilis 534 isolée dans la plaie non suppurée du malade, est caractérisée par des indices morphologiques et les propriétés physiologiques suivants.

Ce sont des bacilles. La grandeur des cellules de la culture d'agar au terme de 24 heures est de (2-4)x-(0,6-0,8) $\mu$m. Bactéries mobiles. Elles forment des spores et ne forment pas de capsules. Elles sont gram-positives. Les colonies sur l'agar de boeuf peptonisé sont rugueuses, aux bords festonnés, de rose faible, d'un diamètre de 2 à 12 mm. La souche se mutiplie à une température de 15 à 50°C, la croissance optimale est à une température de 36 à 37°C. La souche désintègre jusqu'à l'acide, sans dégagement de gaz, le glucose, le saccharose, la mannite. Elle ne fermente pas le lactose, ne forme pas d'hydrogène sulfuré ni d'indole. Elle dégage l'acétylméthylcarbinol et la catalase ; la souche est sensible à la benzylpénicilline, l'ampicilline, l'érythromicyne, la monomicyne, la lincomicyne, la tétracycline, et est insensible à la polymyxine.

La souche produit un antibiotique de large spectre d'action inhibant la croissance des staphylocoques, streptocoques, du protée, de bacillus pyocyaneus et des saccharomycètes. En outre, la souche dégage des enzymes protéolytiques, désintégrant les protides et le modulateur d'immunité.

La nouvelle souche Bacillus sp. 538 est isolée dans l'organisme chez l'homme. Ce sont des bacilles. La grandeur des cellules de la culture d'agar au terme de 24 heures est de (5-10)x(0,7-1,2) $\mu$m. Les bacilles croîssent sous forme de chaîne constituée de 3 à 5 cellules. Les bactéries sont mobiles, gram-positives. Les spores sont disposées de manière subterminale, elles ne forment pas de capsules. Les colonies sur l'agar de boeuf peptonisé sont légèrement convexes, jaunâtres, de 2 à 4 mm de diamètre. Sur le bouillon de boeuf peptonisé, elles croîssent à la surface sous forme de pellicule et sous forme de flocons au fond, sur une solution à 7% de chlorure de sodium dans des conditions aérobie et anaérobie à une température de 25 à 40°C, avec l'optimum à 36-37°C. Elles ne fermentent pas le lactose, le glucose, le saccharose, la mannite, l'arabinose, le xylose. Elles dégagent de l'hydrogène sulfuré, l'indole ne se forme pas. Elles ne coagulent pas le plasma, ne donnent pas d'essai dermonécrotique positif.

La souche est sensible à l'érythromycine, l'oléandomycine, la méthylcilline, la carbenylcilline, la ristomycine, la lévomycétine, la souche est stable à la polymyxine, la lincomycine, la monomycine.

La souche produit une substance antibactérienne de large spectre d'action.

La souche dégage dans le milieu extérieur des enzymes protéolytiques décomposant l'albumine, la fibrine, l'hémoglobine Elle produit des substances modulant l'immunité, réduisant l'allergie.

4

La souche est atoxique et non pathogène.

La nouvelle souche Bacillus pulvifaciens 535 est isolée dans la plaie non suppurée du malade. C'est un bacille dont la grandeur des cellules de la culture d'agar au terme de 24 heures est de (2-4)x(0,6-0,8) $\mu$m. Les bactéries sont mobiles, en croissant sur le bouillon de boeuf peptonisé, forment une pellicule ridée. La souche forme des spores, ne forme pas de capsules, les bactéries sont gram-positives. Les colonies sur l'agar de boeuf peptonisé sont rugueuses, aux bords festonnés, colorées de rose faible, de 2 à 9 mm de diamètre, elles croissent dans des conditions aérobie et anaérobie. Elles se multiplient à une température de 15 à 20°C, avec la croissance optimale à 36-37°C. Elles désintègrent jusqu'à l'acide, sans dégagement de gaz, le glucose, le saccharose, le maltose, le dulcite, le galactose, ne fermentent pas le lactose, la mannite, le xylose, le ramnose, la lysine, l'arginine, l'ornithine. La souche ne forme pas d'hydrogène sulfuré ni d'indole, peptonise le lait. Les bactéries ne produisent pas la lécithinase, elles dégagent l'acétylméthylcarbinol et la catalase, n'hydrolysent pas l'amidon.

La souche est sensible à l'érythromycine, l'ampicilline, la méthylcilline, la benzylpénicilline, l'oxacilline, la lincomycine, la néomycine, la lévomycétine, la monomycine, la ristomycine, la canamycine, la tétracycline, l'oléandomycine ; et est insensible à la polymyxine.

La souche Bacillus pulvifaciens produit des enzymes protéolytiques décomposant l'albumine, l'hémoglobine, la fibrine, un antibiotique de large spectre d'action et le modulateur d'immunité. Les micro-organismes potentiellement pathogènes sensibles à l'antibiotique sont : staphylocoques, streptocoques, colibacilles, bacilles pyocyaniques, protée, salmonelles, bacilles dysentériques, clebsias, saccharomycètes. L'antibiotique n'inhibe pas la croissance de la microflore saprophytique. En outre, l'antibiotique se détruit complètement (rapidement au bout de quelques heures), ce qui exclut la formation d'allergie alimentaire. Les enzymes protéolytiques favorisent l'assimilation du fourrage.

Une différence essentielle de la souche indiquée de Bacillus subtilis 534 est l'absence d'hydrolyse de l'amidon et de désintégration de la mannite.

La souche est atoxique et non pathogène.

La préparation bactérienne selon l'invention fut étudiée dans les conditions de laboratoire sur les animaux et en clinique chez les hommes.

On a également étudié l'antagonisme de la préparation selon l'invention vis-à-vis des agents pathogènes de l'infection bactérienne.

L'antagonisme de la souche Bacillus substilis 534 vis-à-vis des agents pathogènes de l'infection bactérienne fut déterminé par la méthode élaborée par V.I. Nikitenko. On ensemençait dans les éprouvettes contenant 5 cm$^3$ de bouillon de Kolbek stérile 40-50 millions de cellules de culture-test (témoin) et dans les mêmes doses, la culture-test avec la souche indiquée. Les éprouvettes furent incubées au thermostat pendant 24 heures à la température de 37°C. Comme les bactéries de la souche Bacillus substilis 534 modifiaient d'une façon insignifiante la densité du bouillon, le degré d'inhibition de la croissance de la culture-test fut déterminé au moyen du photoélectrocolorimètre d'après la différence de densité du bouillon d'essai et de celui de témoin. On évalua le résultat comme positif, si la densité du mélange était inférieure de 1,2 fois et plus à celle du bouillon de la culture-test.

La souche Bacillus substilis 534 inhibait la croissance de 34 souches sur 37 de staphylocoques étudiées, 11 souches sur 12 de streptocoque, 8 souches sur 12 de colibacille, 7 souches sur 8 de bacille dysentérique, 7 souches sur 7 de salmonelle, 6 souches sur 6 de protée, 7 souches sur 8 de bacille pyocyanique. Les souches de micro-organismes saprophytiques furent essentiellement résistantes et moins sensibles.

Afin de déterminer la production des substances antibactériennes dans le milieu extérieur, les cultures de souche Bacillus subtilis de trois séries de laboratoire furent cultivées durant 96 heures dans des ballons de vibrations sur des balanciers (240 tr/mn) à la température de 28°C sur le milieu nutritif de composition suivante, % en masse : farine de pois : 1,5 ; saccharose : 2,1 ; amidon : 0,85 ; NaNO$_3$ : 0,5 ; CaCO$_3$ : 0,5 ; NaCl : 0,5, eau : complément à 100. L'activité antibactérienne de 0,1 cm$^3$ de filtrat de liquide cultural fut déterminée par la méthode de diffusion dans l'agar à 2% de bouillon de boeuf peptonisé, compte tenu de la grandeur des zones d'inhibition de la croissance de la culture-test avec la soustraction du diamètre des poquets (8 mm).

La croissance du staphylocoque fut inhibée dans les zones de 24-27 mm ; la croissance du colibacille, dans les zones de 18-22 mm ; de la clebsia, dans les zones de 18-21 mm ; des saccharomycètes, dans les zones de 22-24 mm.

Dans les essais in vitro, la souche Bacillus sp. 538 inhibait la croissance de 11 souches sur 12 de staphylocoques étudiées, de 5 souches sur 6 de streptocoques, de 7 souches sur 9 de salmonelles. En cas d'utilisation de la méthode de diffusion dans l'agar contenant les microbes-tests et le liquide cultural, la croissance du staphylocoque fut inhibée dans la zone de 35,1 ± 0,3 mm ; du colibacille, de 22,0 ± 0,2 mm ;

de clebsia, de 20,9 ± 0,2 mm ; des saccharomycètes, de 28,0 ± 0,2 mm. En cas de dilution de 1:10, les zones d'inhibition de la croissance furent, avec le staphylocoque 27,9 ± 0,1 mm ; avec le colibacille 20,0 ± 0,1 mm ; avec la clebsia de 20,0 ± 0,2 mm ; avec les saccharomycètes 29,2 ± 0,2 mm. L'antibiotique brut conservait son activité dans la solution à la température ambiante durant 3 jours.

La souche Bacillus pulvifaciens 535 inhibait la croissance de 34 souches sur 37 de staphylocoque étudiées, 11 souches sur 12 de streptocoques, 12 souches sur 12 de colibacille, 7 souches sur 8 de bacille dysentérique, 7 souches sur 7 de salmonelle, 6 souches sur 6 de protée, 7 souches sur 8 de bacille pyocyanique. Les souches de micro-organismes saprophytiques furent principalement résistantes et moins sensibles. L'antibiotique brut dans la solution fut détruit durant quelques heures.

On a réalisé l'étude de la production des protéases dans le milieu extérieur à partir de la préparation bactérienne selon l'invention.

La production des enzymes protéolytiques dans le milieu extérieur des trois séries de préparation de laboratoire à base de la souche Bacillus subtilis 534 fut déterminée par la méthode d'Anson.

La préparation de chacune des trois séries possède un pouvoir de produire les enzymes décomposant l'alumine, l'hémoglobine, la fibrine. La présence d'enzymes pour d'autres substrats ne fut pas déterminée.

De pair avec les antibiotiques, les souches Bacillus pulvifaciens 535 et Bacillus sp. 538 produisent de façon exogène des enzymes protéolytiques décomposant l'hémoglobine et l'albumine. La présence de protéases fut également déterminée par la méthode d'Anson avec les cultures vivantes de 24 heures.

De même, on a réalisé l'examen de la production de modulateurs d'immunité à partir de la préparation selon l'invention. Le liquide cultural de 72 heures, après élimination des bactéries de souches Bacillus subtilis 534 et Bacillus Sp. 538, en quantité de 0,1 cm$^3$ agit de la même manière que la phytohémagglutinine, à la réaction de transformation en blastes.

On a effectué 6 essais.

Le pourcentage de la formation de blastes dans l'essai témoin est de 0,05%, et après l'introduction du liquide cultural de souche Bacillus subtilis 534 : 42±2%, après l'introduction du liquide cultural de souche Bacillus sp. 538 : 54±2%.

Au cours du traitement avec la préparation bactérienne à base de souches Bacillus subtilis 534, Bacillus pulvifaciens 535, Bacillus sp. 538, E. coli M17, Lactobacillus plantarum 8-PA-3, B.bifidum n° 1, on note l'augmentation du volume des follicules lymphoïdes dans la rate, le foie, l'intestin. Ce fait confirme l'effet immunomodulateur de la préparation.

L'action curative de la préparation selon l'invention à base de souche Bacillus subtilis 534 (3 séries) fut étudiée sur les modèles d'infection expérimentale. Dans ce but, des souris blanches de 19,2±0,1 g de masse furent infectées par voie intrapéritonéale avec une quantité de micro-organismes telle qu'elle entraîna la perte de presque tous les animaux (DL$_{100}$). Le staphylocoque doré fut introduit en dose de 100 à 200 millions de cellules avec 0,8 cm$^3$ d'agar à 0,4% de boeuf peptonisé, les salmonelles en dose de 500 milles à 2 millions avec 0,9 cm$^3$ d'agar de boeuf peptonisé et 100-500 millions de cellules dans 1 cm$^3$ d'une solution à 9% de chlorure de sodium, le bacille pyocyanique en dose de 500 millions de cellules dans 1 cm$^3$ d'une solution à 9% de chlorure de sodium.

La souche Bacillus subtilis 534 fut introduite par voie intrapéritonéale avec de l'eau en dose journalière de 200 millions de cellules durant 10 jours. Les résultats du traitement de l'infection expérimentale sont présentés au tableau 1.

A la suite de l'introduction intrapéritonéale de la préparation, le pourcentage en perte des animaux fut réduit de 89-100% à 25-30% et après l'administration par voie interne, de 92-100% jusqu'à 16,7%. L'activité curative de chacune des trois séries de laboratoire étudiées fut environ identique.

Les résultats de l'étude de la préparation selon l'invention à base des souches Bacillus pulvifaciens 535 et Bacillus sp. 538 sur les modèles analogues d'infection expérimentale, sont présentés au tableau 2.

Le pourcentage en perte des animaux lors du traitement des infections de staphylocoque, de salmonellose et de bacille pyocyanique fut réduit de 100-87,5% (témoin) jusqu'à 40-12,5% (essai) (p < 0,001). A la suite de l'introduction intrapéritonéale de la préparation selon l'invention, en moyenne 28,1% des souris blanches furent perdues et en cas de traitement per os - 17,0% d'animaux.

Tableau 1

Résultats du traitement de l'infection expérimentale chez les
souris blanches par la préparation selon l'invention à base de la
souche Bacillus subtilis 534

| n° | Genre de l'infection | Nombre de souris | Témoins | Traitement | |
|---|---|---|---|---|---|
| | | | Nombre en % d'animaux perdus | I série | |
| | | | | Nombre de souris | Souris perdues |
| 1 | 2 | 3 | 4 | 5 | 6 |
| 1. | Staphylocoque (introduction intrapéritonéale de la préparation) | 20 | 18(90%) | 7 | 2 |
| 2. | Salmonellose (introduction intrapéritonéale de la préparation) | 36 | 32(89%) | 12 | 4 |
| 3. | Bacille pyocyanique (introduction intra- péritonéale de la préparation) | 10 | 10(100%) | 4 | 2 |
| 4. | Staphylocoque (introduction par voie interne de la préparation) | 24 | 22(92%) | 8 | 1 |
| 5. | Salmonellose (introduction par voie interne de la préparation) | 18 | 18(100%) | 6 | 0 |

EP 0 363 491 B1

Tableau 1 (suite)

| n° | Traitement | | | | | |
|----|------------|------|------------|------|------------|------|
| | 2ème série | | 3ème série | | 4ème série | |
| | Nombre de souris | Souris péries | Nombre de souris | Souris péries | Nombre de souris | Souris péries |
| | 7 | 8 | 9 | 10 | 11 | 12 |
| 1. | 7 | 2 | 6 | 2 | 20 | 6(30%) |
| 2. | 12 | 2 | 12 | 3 | 36 | 9(25%) |
| 3. | 3 | 0 | 3 | 1 | 10 | 3(30%) |
| 4. | 8 | 2 | 8 | 1 | 23 | 4(16,7%) |
| 5. | 6 | 2 | 6 | 1 | 18 | 3(16,6%) |

Tableau 2

Résultats du traitement de l'infection expérimentale chez les souris
par la préparation selon l'invention, à base de souches
Bacillus pulvifaciens 535 et Bacillus sp. 538

| n° | Préparation | Infection de staphylocoque | |
| --- | --- | --- | --- |
| | | Quantité d'animaux | Animaux péris (nombre,%) |
| 1 | 2 | 3 | 4 |
| 1. | Groupe de témoins | 20 | 18(90%) |
| 2. | Groupe d'essai à l'introduction intrapéritonéale de la préparation à base de souche Bacillus pulvifaciens 535 | 20 | 5(25%) |
| 3. | Groupe de témoins | 8 | 7(87,5%) |
| 4. | Groupe d'essai à l'introduction intrapéritonéale de la préparation selon l'invention à base de souche Bacillus sp. 538 | 8 | 2(25%) |
| 5. | Groupe de témoins | 8 | 7(87,5%) |
| 6. | Groupe d'essai à l'introduction de la préparation selon l'invention à base de souche Bacillus pulvifaciens 535 | 8 | 2(25%) |
| 7. | Groupe de témoins | 8 | 8(100%) |
| 8. | Groupe d'essai à l'introduction de la préparation selon l'invention à base de Bacillus sp. 538 | 8 | 1(25%) |

EP 0 363 491 B1

Tableau 2 (suite)

| n° | Salmonellose | | Bacille pyocyanique | |
|---|---|---|---|---|
| | Quantité d'animaux | Animaux péris (nombre, %) | Quantité d'animaux | Animaux péris (nombre, %) |
| 1 | 5 | 6 | 7 | 8 |
| 1. | 36 | 32(88,9%) | 10 | 9(90%) |
| 2. | 36 | 9(25%) | 10 | 4(40%) |
| 3. | 5 | 5(100%) | 10 | 10(100%) |
| 4. | 5 | 1(20%) | 10 | 4(40%) |
| 5. | 18 | 16(88,9%) | | |
| 6. | 18 | 4(22,2%) | | |
| 7. | 13 | 13(100%) | | |
| 8. | 13 | 2(15,4%) | | |

La salmonelle en dose de 2 milliards de cellules dans 1 cm$^3$ d'une solution à 9% de chlorure de sodium fut introduite dans 12 rats de lignée Wistar. 6 animaux recevaient durant 7 jours la préparation selon l'invention par voie intrapéritonéale en dose journalière de 500 millions de cellules de la culture vivante de souche Bacillus pulvifaciens 535. Dans le groupe de témoins, tous les animaux ont péri, dans le groupe d'essai (de traitement) n'a péri qu'un rat.

A 12 lapins d'un poids de 1,5-2,2 kg, on fit 4 plaies linéaires (jusqu'aux muscles) sur leur dos. Les bords de chaque plaie furent infiltrés avec 1 ml de 5 milliards de cellules en suspension de la culture vivante de 24 heures de staphylocoque doré isolé de la plaie suppurée du malade, 6 animaux recevaient par jour, avec la nourriture, 1 milliard de cellules de la préparation selon l'invention obtenue à base de la souche Bacillus subtilis 534. En comparaison avec les lapins témoins, chez les lapins traités par la préparation bactérienne, on nota une dimension des infiltrats inflammatoires de 2 à 2,8 fois plus petite. Au dixième jour, la surface de la peau nécrosée fut en moyenne de 5,7 cm$^2$ chez les animaux témoins et de 1,3 cm$^2$ chez les animaux traites.

Des plaies se cicatrisant furent examinées par l'analyse histologique au 4ème et au 12ème jour. Les coupes histologiques furent colorées avec l'hématoxyline-éosine. Dans les coupes histologiques prises au 4ème jour, il n'y avait pas de différences sensibles. Au 12ème jour, dans les plaies des lapins traités, en comparaison avec les lapins témoins, les granulations étaient plus mûres, l'anisomorphotropie verticale y disparut, elles avaient perdu leur structure couche par couche. On nota une épidermisation plus intense, une couche d'épithélium plus large. De pair avec les observations cliniques et les examens histologiques, furent réalisés les ensemencements de la peau, du sang et des plaies. Après l'isolement de la culture pure du genre Bacillus, furent étudiées leurs propriétés physiologiques, tinctoriales, biochimiques et antigéniques. On ne trouva pas la souche Bacillus subtilis 534 sur la peau. Les résultats de la découverte de la souche Bacillus subtilis 534 dans le sang et les plaies sont donnés dans le tableau 3.

Tableau 3

| Résultats du décèlement des bactéries de la souche Bacillus subtilis 534 | | | | | | |
|---|---|---|---|---|---|---|
| | Quantité d'animaux chez qui ont été isolées les bactéries de la souche Bacillus subtilis 534 | | | | | |
| | Après 24 H | | Après 4 jours | | Après 12 jours | |
| | plaie | sang | plaie | sang | plaie | sang |
| 1 | 2 | 3 | 4 | 5 | 6 | 7 |
| Groupe de 6 témoins | 0 | 0 | 0 | 0 | 0 | 0 |
| Groupe de 6 animaux traités (d'essai) | 2 | 6 | 6 | 3 | 6 | 1 |

Afin d'étudier l'effet curatif des trois séries de laboratoire de la préparation selon l'invention à base de la souche Bacillus subtilis 534, les expériences furent réalisées sur les souris blanches auxquelles on avait fait avec le scalpel, des plaies linéaires d'environ 8 mm (jusqu'aux muscles). Les bords des plaies furent infiltrés avec 0,25 cm$^3$ d'une solution à 0,9% de chlorure de sodium contenant 2 milliards de cellules de la culture vivante de 24 heures de staphylocoque doré isolé dans la plaie suppurée du malade. Le groupe de témoins fut constitué de 18 mâles et femelles. 24 souris recevaient tous les jours 200 millions de cellules de souche indiquée, par voie interne.

Dans le groupe de témoins, 2 à 6 jours après, 7 souris furent perdues (d'après les données de l'autopsie - de la septicémie). Tous les animaux du groupe d'essai furent vivants. Ils furent sacrifiés au 4ème et 12ème jour. A la suite de l'examen histologique du foie, de la rate au 4ème et au 12ème jour et des tissus de la plaie au 4ème jour, on n'avait pas trouvé de différences entre les animaux des groupes d'essai et des témoins. Au 12ème jour, chez 11 souris sur 15 traitées par la préparation selon l'invention, on observa l'épithélisation, tandis que les animaux témoins n'eurent que leurs éléments initiaux.

Au dos des 12 lapins d'un poids de 1,5 à 2,2 kg, on fit 4 plaies linéaires (jusqu'aux muscles). Les bords de chaque plaie furent infiltrés avec 1 ml de suspension de 5 milliards de cellules de la culture vivante de staphylocoque doré isolé dans la plaie suppurée du malade. 6 animaux recevaient par jour avec la nourriture, la préparation selon l'invention contenant 1 milliard de cellules de la culture lyophilisée vivante de souche Bacillus pulvifaciens 535. Par rapport aux lapins témoins, chez les lapins traités par la préparation bactérienne selon l'invention, durant les 5 premiers jours, on nota des dimensions des infiltrats inflammatoires de 1,9-2,8 fois plus petites. Au 10ème jour, les animaux témoins avaient la surface de la peau nécrosée d'environ 5,8 cm$^2$ et les animaux d'essai - de 1,5 cm$^2$. Chez les animaux témoins, se développèrent des processus de suppuration marqués avec une grande quantité de secrétion, tandis qu'à la suite du traitement par la préparation selon l'invention, la cicatrisation se fit sous la croûte sèche.

Lors de l'observation des animaux, furent effectués les ensemencements du sang prélevé de la veine de l'oreille et de la plaie sur une solution à 5% de glucose. Au 4ème et 12ème jour, on avait traité deux fois la plaie par une solution alcoolique de chlorhexidine et disséqué avec un scalpel stérile. Les ensemencements furent pris à partir de la plaie profonde. Les résultats des examens bactériologiques sont présentés au tableau 4.

Les examens des propriétés morphologiques, tinctoriales, biochimiques et antigéniques des bactéries et de leur sensibilité aux antibiotiques ont montré que du sang et de la plaie des lapins témoins, on isolait les bactéries de souche Bacillus pulvifaciens 535.

Tableau 4

| Résultats de l'isolement des bactéries de la souche Bacillus pulvifaciens 535 dans la plaie infectée expérimentalement, chez des lapins | | | | | | |
|---|---|---|---|---|---|---|
| | Quantité d'animaux chez lesquels ont été isolées les bactéries de la souche Bacillus pulvifaciens 535 | | | | | |
| | Après 24 H | | Après 4 jours | | Après 12 jours | |
| | plaie | sang | plaie | sang | plaie | sang |
| 1 | 2 | 3 | 4 | 5 | 6 | 7 |
| Groupe témoin (11 animaux) | 0 | 0 | 0 | 0 | 0 | 0 |
| Groupe d'essai (12 animaux) | 5 | 10 | 11 | 7 | 11 | 4 |

Au dos des 8 lapins d'un poids de 1,5-2,2 kg, on fit des plaies linéaires de 3 cm de longueur. Les bords de chaque plaie furent infiltrés avec 5 milliards de cellules de staphylocoque doré contenues dans 1 cm$^3$ d'une solution à 0,9% de chlorure de sodium. Les plaies furent recouvertes de colle médicale. Pour le traitement, on introduisait aux 4 lapins une fois par 24 heures la préparation selon l'invention à base de souche B. bifidum n° 1 en quantité de 200-500 millions de cellules vivantes. 10 jours après, la surface des plaies chez les animaux témoins fut de 6,1±0,3 cm$^2$, et celle des animaux traités par la préparation selon l'invention : de 3,4 ±0,2 cm$^2$.

La souche B. bifidum n° 1 fut isolée dans une période de 0,5 a 24 heures chez 2 lapins, dans une période de 48 à 72 heures à nouveau chez 2 lapins qui recevaient la préparation. La souche fut isolée dans la plaie chez 3 lapins.

On a réalisé les études du mécanisme d'action de la préparation selon l'invention à l'aide de l'historadiographie.

Dans les éprouvettes contenant l'agar de boeuf peptonisé, avec 1 cm$^3$ de leucine marquée au 14$_C$ - (dose de 1 micron par éprouvette), on fit croître pendant 48 heures, à la température de 37° C, les souches Bacillus subtilis 534 et Bacillus sp. 538.

Pour l'essai, on avait pris 8 lapins d'un poids de 2-2,5 kg. A 4 animaux, on fit sous la narcose d'ingalation, dans la région du dos, des plaies sur la peau de 4 cm de longueur. Les plaies furent cousues avec un fil de soie et recouvertes d'une couche de colle médicale.

Les micro-organismes cultivés furent introduits par voie intragastrique au moyen d'une sonde stomacale, à chaque lapin en dose de 1 milliard de cellules dans 10 cm$^3$ d'une solution à 0,9% de chlorure de sodium, au début de l'essai et ensuite au bout de 24 heures.

De la veine de l'oreille, on a prélevé le sang 24 et 48 heures après l'introduction des micro-organismes. Au bout de 48 heures, les lapins furent décapités. On avait pris pour les examens historadiographiques les tissus de la plaie, de la peau (chez les lapins sans plaies), du foie, de la rate, de l'estomac, du gros intestion et de l'intestin grêle, etc. Les matériaux furent placés dans la solution à 10% de formaline. Ultérieurement, on réalisa des coupes, l'application de la photo-émulsion avec l'exposition durant un mois, la coloration des coupes et les prises de vues. On enregistra chez tous les animaux l'apparition des bactéries dans le sang, 24 heures après l'introduction. En outre, les bactéries furent décelées dans les follicules lymphoïdes de l'intestin grêle et dans le tissu conjonctif dans la région des plaies. Les bactéries se multipliaient dans les tissus autour de la plaie. Cela confirme la présence de chaînes de bactéries. On ne trouva pas de bactéries dans le tissu conjonctif de la peau des lapins sans plaies. Les bactéries peuvent pénétrer et se conserver dans d'autres tissus, par exemple, dans la rate. Il est important de ne pas avoir décelé de modifications pathologiques dans les coupes histologiques des tissus, malgré la présence de bactéries.

Les résultats des études réalisées précédemment sont évidemment confirmés. Les bactéries d'une même souche peuvent pénétrer de l'intestion grêle, avec le courant de sang, dans la plaie. Elles peuvent pénétrer dans la plaie, avec le courant de sang, à partir des autres sources endogènes, par exemple, de la rate.

Pour déterminer la toxicité aiguë de la préparation selon l'invention, on avait utilisé 3 séries de la préparation obtenue à base de culture vivante de la souche Bacillus subtilis 534. Chaque série fut introduite

EP 0 363 491 B1

unen fois par voie intrapéritonéale à 3 souris blanches et à 3 rats de lignée Wistar respectivement en doses de 10 et 20 milliards de cellules dans 1 cm$^3$ d'une solution à 0,9% de chlorure de sodium. Tous les animaux restèrent vivants. Ils mangeaient bien la nourriture. Les animaux furent sacrifiés 3,7, 14 jours après l'essai. L'étude histologique du cerveau, des poumons, des reins, du foie et du coeur ne montra pas de modifications inflammatoires, ni de dystrophies.On nota dans la rate une augmentation des dimensions des follicules et de la quantité d'histiocytes lymphocytaires dans la pulpe splénique.

Afin d'étudier le degré de toxicité aiguë, les trois séries de préparation de souche Bacillus subtilis 534 furent introduites chacune une fois avec la nourriture dans 5 souris blanches et 6 rats blancs de lignée Wistar respectivement à des doses de 10 et 20 milliards de cellules. Tous les animaux restèrent vivants. Le jour suivant, les animaux furent sacrifiés. A la suite des examens histologiques du cerveau, du myocarde, des poumons, des reins, de l'estomac, de l'intestin grêle et du gros intestin, on n'avait pas trouvé de modifications en comparaison avec les animaux témoins (10 souris et 10 rats). Dans le fois, on nota une certaine augmentation de la quantité d'infiltrats histio-lymphocytaires dans le système porte-hépatique. Dans le rat, on décela l'augmentation des dimensions des follicules, une forte quantité d'histiocytes lymphocytaires dans la pulpe splénique et de cellules de Sterberg.

A 20 souris blanches, furent introduites par voie intrapéritonéale, les souches Bacillus pulvifaciens 535 à Bacillus sp. 538 préalablement tuées avec les vapeurs de formaline en dose de 2 milliards de cellules dans 1 cm$^3$ d'une solution à 0,9% de chlorure de sodium. De nouveau à 30 souris et à 30 rats blancs de lignée Wistar, furent introduites des cultures vivantes de ces mêmes souches en doses de 10 et 20 milliards de cellules. Tous les animaux restèrent vivants. Ils mangeaient bien la nourriture. Les animaux furent sacrifiés 37, 14 jours après l'essai. Lors de l'étude histologique du cerveau, des poumons, des reins, du coeur, du foie, on ne trouva pas de modifications inflammatoires et de dystrophies. On nota dans la rate l'augmentation des dimensions des follicules et de la quantité d'histiocytes lymphocytaires dans la pulpe splénique.

A 15 souris blanches et à 15 rats blancs de lignée Wistar, furent introduits per os respectivement 10 et 20 milliards de cellules en suspension d'une culture lyophilisée vivante de la souche Bacillus pulvifaciens 535. Tous les animaux restèrent vivants. Le lendemain, ils furent sacrifiés. Lors de l'étude histologique du cerveau, du myocarde, des poumons, des reins, de l'estomac, de l'intestion grêle et du gros intestin, on ne décela pas de modifications, en comparaison avec les animaux témoins. On nota dans le foie une certaine augmentation de la quantité d'infiltrats histiolymphocytaires dans le système porte-hépatique. Dans la rate, on décela l'augmentation des dimensions des follicules et une grande quantité d'histiocytes lymphocytaires dans la pulpe splénique, et de cellules de Sterberg.

A 12 lapins, des cultures vivantes de 24 heures des souches Bacillus pulvifaciens 535 et Baciullus sp.538, furent introduites par la voie sous-cutanée, à chacun à des doses de 100 milles, 1 million, 10 millions, 1 milliard, 5 milliards de cellules dans 1 cm$^3$ d'une solution à 0,9% de chlorure de sodium. L'état général des animaux ne fut pas changé, tous les animaux restèrent vivants. On n'observa pas de modifications locales à l'endroit d'introduction, mais on décela dans la zone d'infection, durant 2 semaines, des bactéries sporifères.

Pour la détermination de la toxicité chronique, les trois séries de la préparation à base de la souche Bacillus subtilis 534 furent introduites chacune durant 30 jours par voie intrapéritonéale dans 3 souris blanches et dans 3 rats blancs de lignée Wistar respectivement à des doses de 20 millions et de 1 milliard de cellules dans 1 ml d'une solution à 0,9% de chlorure de sodium. En outre, chacune des trois séries de la préparation fut introduite dans des doses équivalentes dans 6 souris blanches et dans 6 rats blancs de lignée Wistar. Tous les animaux restèrent vivants. La masse des animaux dans le groupe d'essai, en comparaison avec celle des animaux témoins (par 10 souris et rats), fut supérieure de 11-17% (p < 0,05). Le poil chez les souris et les rats dans le groupe d'essai fut de blanc vif, duveteux. Les animaux furent sacrifiés au 31ème jour. A la suite de l'examen histologique du cerveau, du myocarde, des reins, des poumons, de l'estomac, de l'intestin grêle et du gros intestin, on ne décela pas de modifications. Dans le foie, il y avait en grande quantité, en comparaison avec les animaux témoins, des infiltrats histiolymphocytaires disposés dans le système porte-hépatique. Dans la rate des animaux, dans le groupe d'essai, la lymphoïdisation de la pulpe splénique et l'augmentation des dimensions des follicules furent notées, par contre, on ne décela pas de cellules de Sterberg.

Lors de la réalisation des essais portant sur l'étude de la toxicité chronique après 11-21 jours, 6 souris blanches avaient mis au monde 23 souriceaux sans monstre. Ultérieurement, ces dernières avaient mis au monde 7 souriceaux sans monstre.

Pour déterminer la toxicité chronique, les cultures vivantes de 24 heures des souches Bacillus pulvifaciens 535 et Bacillus sp. 538 étaient introduites durant 30 jours par voie intrapéritonéale à 40 souris blanches en dose journalières de 200-500 millions de cellules et à 30 rats de lignée Wistar en dose

EP 0 363 491 B1

journalière de 2 milliards de cellules dans 1 cm$^3$ d'une solution à 0,9% de chlorure de sodium. Outre cela, 48 souris blanches et 30 rats de lignée Wistar recevaient avec de l'eau durant 30 jours, 200 millions et 1 milliard de cellules de culture lyophilisée des souches indiquées par jour. Tous les animaux restèrent vivants. Soulignons que le poids des animaux dans le groupe d'essai, en comparaison avec celle des animaux témoins, augmenta de 11,19% et au-delà. Le poil chez les animaux dans le groupe d'essai fut de blanc vif et duveteux. Les animaux furent sacrifiés. A la suite de l'examen histologique du cerveau, du myocarde, des reins, de l'estomac, de l'intestin grêle et du gros intestin, on ne décela pas de modifications. On décela dans le foie une grande quantité, en comparaison avec les animaux témoins, d'infiltrats histiolymphocytaires disposés dans le système porte-hépatique ou en dehors. Il fut noté que dans la rate des animaux témoins, la lymphoïdisation de la pulpe splénique et l'augmentation des dimensions des follicules eurent lieu, par contre, on ne décela pas de cellules de Sterberg.

Au cours de la réalisation des essais portant sur l'étude de toxicité chronique après 11-21 jours, 9 souris blanches avaient mis au monde 54 souriceaux sans monstre. Ces dernières avaient mis au monde 9 souriceaux sans monstre. De cette façon, les cultures vivantes des souches, même en cas d'introduction intrapéritonéale, n'influent pas sur le développement du foetus et ne provoquent pas de perturbations génétiques.

Les trois séries de la préparation à base de la souche Bacillus subtilis 534 étaient introduites chacune dans 4 cobayes à des doses de 2 milliards de cellules dans 1 cm$^3$ d'une solution à 0,9% de chlorure de sodium 6 fois par voie sous-cutanée, tous les deux jours. 31 jours passés, on fit, à tous les cobayes, des injections intramusculaires de la préparation à des doses de 2 milliards de cellules dans 1 cm$^3$ d'une solution à 0,9% de chlorure de sodium. On n'a pas noté de manifestations locales et générales des réactions allergiques.

Les cultures vivantes des souches Bacillus pulvifaciens 535 et Bacillus sp. 538 à des doses de 2 milliards de cellules dans 1 ml d'une solution à 0,9% de chlorure de sodium, furent introduites 6 fois tous les deux jours par voie sous-cutanée à 24 cobayes (une souche pour 12 animaux). 31 jours après, on fit à tous les cobayes des injections sous-cutanées en dose de 2 milliards de cellules des micro-organismes de ces mêmes souches dans 1 cm$^3$ d'une solution à 0,9% de chlorure de sodium. On ne décela pas de manifestations locales et générales des réactions allergiques.

Pour déterminer la stabilité (la résistance) de la préparation selon l'invention, 9 capsules de préparation obtenue à base des souches Bacillus subtilis 534, Bacillus sp. 538, Bacillus pulvifaciens 535 en flacons étanches furent stockées à température ambiante durant 7 ans. A nouveau, 9 capsules furent stockées pendant 3 mois à une température de moins de 20-22°C, 9 capsules furent stockées durant 2 mois à la température de 110°C. La quantité de bactéries vivantes dans les capsules fut déterminée par la méthode d'ensemencement des séries diluées.

Avant le début des essais, la teneur en bactéries de la capsule fut de 5,4±0,5 milliards de cellules, après le stockage de 7 ans de 5,2±0,5 milliards de cellules, après le stockage à une température de moins de 20-22°C, de 5,4±0,6 milliards de cellules, après le stockage à la température de 110°C, de 5,3±0,6 milliards de cellules. De cette façon, les données obtenues témoignent de ce que la préparation selon l'invention peut se conserver jusqu'à 7 ans dans une large gamme de températures.

Différentes versions de la préparation bactérienne selon l'invention ont été utilisées en clinique pour la prophylaxie et le traitement des processus purulent inflammatoires, des affections allergiques, des perturbations de digestion, des dysbactérioses chez 117 malades.

Dans le but de prévenir la purulence, on a utilisé la préparation obtenue à base de la souche Bacillus subtilis 534 à des doses de 50-500 milles cellules après un traitement chirurgical primaire chez 8 malades avec des fractures ouvertes des extrémités et chez 5 malades avec des lésions accidentelles des tissus musculaires. La suppuration se développa seulement chez un malade. Pour le traitement local des processus purulents, la préparation selon l'invention, dans les mêmes doses étant utilisée localement durant 10 jours chez 3 malades avec une ostéomyélite post-traumatique aiguë chez 2 malades avec des plaies des tissus musculaires et chez 2 malades avec l'inflammation à l'endroit des broches de l'appareil d'Ilizarov. La suppuration s'était arrêtée sans emploi des antiseptiques chez 5 malades. Il n'y avait pas de complications.

De pair avec l'effet positif, on a décelé l'effet négatif de l'utilisation locale de la préparation selon l'invention. Avec ce procédé d'utilisation, les phénomènes tels que la lymphadénite, la lymphangite, la thrombophlébite sont conservés, et des complications lors du traitement des autres processus inflammatoires, par exemple, des pneumonies surgissent. De ce fait, on a refusé l'utilisation locale de la préparation selon l'invention et on a réalisé les essais par administration par voie interne.

Pour prévenir l'infection purulente chirurgicale, la préparation était administrée par voie interne à des doses de 5 milliards de cellules par 24 heures durant 10 jours à 9 malades avec les fractures ouvertes des extrémités, à 7 malades avec des plaies accidentelles des tissus musculaires, à 12 malades après des interventions opératoires (ostéosynthèse). Pour le traitement, la préparation selon l'invention fut introduite à des doses de 1-10 milliards de cellules, 1 à 2 fois par jour par voie interne pendant 10 jours chez 14 malades souffrant de l'ostéomyélite aiguë et chronique, chez 14 malades ayant des plaies purulentes, chez 6 malades ayant des processus purulent-inflammatoires dans la région des broches des appareils à compression-dilatation, chez un malade ayant un trauma cérébral et une pneumonie hypostatique, chez un malade ayant un trauma cérébral, une pneumonie, une méningite, des escarres de décubitus, chez 1 malade ayant un sepcis chronique dû au diabète sucré, chez 2 malades ayant une amygdalite, chez 1 malade ayant de la sinusite. D'après les indications, pour 12 malades avec des processus purulents amples, le traitement à partir de la préparation selon l'invention a été répété pendant 3 à 7 jours.

Signalons que 9 malades qui recevaient auparavant les antibiotiques, avaient les manifestations cliniques de dysbactériose : mal-au-ventre et colique abdominale, alternance des constipations et de défécation liquide.

En outre, 7 malades étaient intolérants à un ou plusieurs antibiotiques, à des préparations sulfanylamides, un malade avait des réactions allergiques provoquées par le poisson, les citronniers, la carotte, les tomates.

Au cours du traitement par la préparation selon l'invention, tous les malades ne recevaient pas d'antibiotiques, ni d'autres préparations antiseptiques. Pour le lavage des plaies et les pansements, on utilisait une solution à 10% de chlorure de sodium, une solution à 40% de glucose et une solution à 30% d'urée.

De pair avec l'examen clinique et l'observation, on a réalisé la radiographie de l'extrémité lésionée, la radioscopie et la radiographie du thorax, la thermométrie, les analyses générales du sang et de l'urine, la détermination de l'azote résiduel, de la bilirubine, du sucre et de la quantité de protéines du sang. En outre, on étudiait l'activité bactéricide du sérum du sang, sa teneur en lysozyme, béta-lysine, on isolait dans les plaies et dans le sang les ensemencements avec une détermination subséquente de la composition de la microflore, de ses propriétés physiologiques, biochimiques, antigéniques et de sa sensibilité aux antibiotiques.

En cas d'administration par voie interne de la préparation selon l'invention, la suppuration de la plaie ne fut notée que chez un malade parmi 28. Chez ce malade, avec la fracture ouverte de la jambe à la suite de la néorose de la peau, s'était formée la croûte qui s'exfoliait successivement. La plaie s'était cicatrisée avec une tension secondaire. Chez les malades ne recevant pas la préparation selon l'invention, les processus purulent-inflammatoires étaient plus fréquents : suppuration des fractures ouvertes dans 36% des cas, des plaies accidentelles des tissus musculaires dans 14% des cas, des plaies post-opératoires après l'ostéosynthèse dans 7% des cas.

La dynamique de la température du corps était la suivante : 1 jour 38,1±0,2°C ; 72 heures 37,1±0,1°C ; 7 jours 36,7±0,1°C ; 11 jours 36,4±0,1°C.

Les analyses de sang avant le début du traitement avec la préparation selon l'invention : hémoglobine 101±11 g/l, nombre de leucocytes 8,9±0,4 g/l, VSE 11±0,4 mm/h, éosinocytes 0,9±0,1%, protéine générale 56,1±0,8 g/l ; bilirubine 15,4±0,3 mmol/l ; sucre 4,1±0,1 mmol/1 ; azote résiduel 19,9±0,4 mmol/l. Vers le 11ème jour après l'achèvement du cours de la thérapie prophylactique, le nombre de leucocytes (6,8±0,2 g/l) et VSE (7,2±0,3 mm/h) avait diminué (p < 0,01), la quantité de protéine générale (68±0,6 g/l) et d'hémoglobine (139±8 g/l) avait augmenté (p < 0,05) et le pourcentage d'éosinocytes (0,9±0,1), la quantité de bilirubine (13,9±0,2 mmol/l), de sucre (3,9±0,1 mmol/l), de l'azote résiduel (20,2±0,3 mmol/l) changeaient peu et ne différaient pas des indices des hommes sains. L'activité bactéricide du sérum sanguin était environ stable : 88,2±1,9% et 86,4±1,8%. La teneur en lisozyme du sérum sanguin avait diminué de 7,9±0,5 mg/ml par 24 heures jusqu'à 5,4±0,7 mg/l vers le 11ème jour (p < 0,05). La dynamique des béta-lysines fut analogue : 52,1±2,1% et 44,6±1,8% (p < 0,05). De telles oscillations des indices de résistance naturelle sont caractéristiques du cours non compliqué du trauma. Avant le début du traitement dans les analyses de l'urine des 7 malades, fut établie l'augmentation de la quantité de leucocytes et l'apparition des érythrocytes. Vers le 11ème jour, les analyses de l'urine furent normales.

La souche Bacillus subtilis 534 fut isolée dans le sang chez 27 malades parmi 28 et dans la plaie chez 26 malades parmi 28. On n'avait pas noté de complications après l'utilisation prophylactique de la préparation selon l'invention, ce fait fut confirmé par les observations cliniques et les données d'analyses.

Parmi 36 malades avec des plaies suppurées (y compris avec l'ostéomyélite et la septicémie chronique) le staphylocoque dans la monoculture et la composition d'associations microbiennes, fut isolé chez 26 malades, le bacille pyocyanique chez 6 malades, le colibacille chez 4 malades, le protée chez 3, d'autres bactéries chez 8 malades. 76% des souches obtenues étaient résistantes à l'action de 5 et plus d'antibiotiques.

15

24 heures après le début du traitement, on observa une augmentation sensible de secrétion purulente. Les plaies se libéraient vite des tissus nécrosés. Ultérieurement, le caractère du secteur de la plaie se modifiait, la quantité de secrétion purulente diminuait, elle devenait successivement séro-purulente avec une légère impureté de sang décomposé. 1 à 3 jours après le début du traitement s'était formé le tissu de granulations ; l'oedème, la lymphadénite, la lymphangite avaient disparu, l'épithélisation s'était activée.

La cicatrisation des plaies suppurées eut lieu chez 23 malades parmi 36, chez 11 malades, on nota l'apaisement du processus inflammatoire (normalisation de la température du corps, la purification des plaies des tissus nécrosés, diminution de la quantité de secrétion purulente, etc). Les jours suivants, les plaies s'étaient cicatrisées, les fistules s'étaient fermées chez 9 malades parmi 11. Le traitement par la préparation bactérienne n'eut pas de succès seulement chez 2 malades (aggravation de l'ostéomyélite chronique et suppuration à l'endroit de la broche de l'appareil d'Ilizarov). Chez ce dernier malade, la suppuration à l'endroit d'une broche s'apaisait, mais elle surgissait à l'endroit de l'autre broche. A la suite du traitement des pneumonies, de la méningite, de la septicémie chronique, de l'amygdalite, de la sinusite, tous les malades étaient complètement guéris.

On n'observera pas de réactions allergiques malgré la disposition à celles-ci de certains malades. Au bout de 1 à 4 jours, les manifestations cliniques de la dysbactériose s'étaient apaisées chez les 9 malades. Compte tenu de ce que 21 malades avant l'utilisation de la préparation selon l'invention étaient traités sans effet positif durant 6 à 42 jours par les enzymes protéolytiques, les antibiotiques et d'autres antiseptiques, les résultats obtenus doivent être reconnus pour positifs.

La dynamique de la température corporelle chez les malades avec les processus purulents inflammatoires fut la suivante : avant le traitement, 38,3±0,3°C, au 3ème jour 37,6±0,3°C, au 7ème jour 37,0±0,2°C, au 11ème jour 36,8±0,1°C. Analyses de sang avant le traitement : hémoglobine 122±13 g/l, quantité de leucocytes 9,1±0,5% g/l, VSE 16±0,9 mm/h, éosinocytes 1,5±0,2%, protéine générale 52,4±3,1 g/l, la bilirubine 18,6±0,4 mmol/l, sucre 4,6±0,1 mmol/l, azote résiduel 17,8±0,6 mmol/l. Après l'achèvement du cours du traitement, la quantité de leucocytes (6,9±0,4 g/l) et VSE (9,2±0,8 mm/h) fut réduite (p < 0,001), la teneur en protéine générale augmenta jusqu'à 72±4,4 g/l (p < 0,001). Le pourcentage d'éosinocytes (1,0±0,2%), la quantité d'hémoglobine (138±9 g/l), de bilirubine (16,9±0,3 mmol/l), de sucre (4,8±0,2 mmol/l), d'azote résiduel (17,4±0,6 mmol/l) restèrent dans les limites de la norme.

L'activité bactéricide du sérum sanguin augmenta de 72,7±2,8% jusqu'à 84,6±2,1% (p < 0,01), la teneur en lysozyme, en béta-lysine diminua respectivement de 8,6±0,6 mg/l jusqu'à 6,9±0,5 mg/l et de 57,1±2,4% jusqu'à 47,8±2,7% (p < 0,05) ce qui témoigne de l'apaisement du processus inflammatoire.

Avant le traitement par la préparation bactérienne selon l'invention, on nota dans les analyses de l'urine chez 12 malades l'augmentation de la quantité de leucocytes, l'apparition de la protéine et des cylindres. Vers le 11ème jour, les analyses de l'urine chez 11 malades parmi 12 furent normales. La souche Bacillus subtilis 534 fut isolée dans le sang chez 38 malades parmi 40 et dans la plaie chez 34 malades parmi 36 chez lesquels furent réalisées les études données.

La préparation bactérienne obtenue à base de la souche Bacillus sp. 538 fut utilisée pour le traitement de l'eczéma et la diathèse chez 5 enfants, des processus purulents des extrémités chez 9 malades. A la suite des examens de contrôle, la souche fut isolée dans le sang chez tous les enfants 0,5 à 48 heures après la première administration par voie interne. On réalisa la biopsie de la peau à l'endroit de la lésion avec les examens bactériologiques. La souche fut isolée de la peau après 2 à 4 jours chez 4 malades parmi 5. Tous les enfants étaient guéris. Chez 7 malades parmi 9, le processus purulent fut arrêté sans le traitement supplémentaire.

La préparation selon l'invention à base de la souche E. coli M17 à des doses de 1-5 milliards de cellules fut administrée 2 fois par jour par voie interne à 6 malades ayant des processus purulents dans les tissus musculaires des extrémités. Les pansements furent effectués avec une solution à 10% de chlorure de sodium. Ils ne recevaient pas d'autres préparations antibactériennes. 2 jours après le début du traitement, on nota la baisse de la température et la diminution de secrétion purulente chez 4 hommes parmi 6. A 2 malades sans symptômes cliniques favorables, on administra les antibiotiques.

A l'examen bactériologique du sang avant le début du traitement, les ensemencements du sang furent stériles. 1 à 2 jours après, la souche E. coli M17 fut isolée dans le sang chez 5 malades parmi 6.

Avant le traitement, fut isolé dans la plaie : le staphylocoque doré, dans la monoculture et dans les associations avec le bacille pyocyanique, le protée (2 observations). Le colibacille fut isolé dans la secrétion des plaies avec le staphylocoque à l'issue de 2 à 5 jours du traitement chez 4 malades parmi 6. L'identification montra que ce fut la souche E. coli M17.

La préparation bactérienne selon l'invention, obtenue à base des souches Bacillus subtilis 534, Bacillus pulvifaciens 535, Bacillus sp. 538 fut utilisée pour le traitement de la pyélonéphrite chez 2 malades. La préparation fut administrée par voie interne à des doses de 1 à 10 milliards de cellules par 24 heures durant 7 à 10 jours en 1 à 3 fois. La rémission de l'affection fut marquée chez deux malades.

La préparation selon l'invention fut également essayée en médecine vétérinaire.

La préparation obtenue à base de la souche Bacillus pulvifaciens 535 fut utilisée pour la prophylaxie et le traitement des dyspepsies, des maladies d'oedème des mastites. Pour la prophylaxie, elle fut utilisée avec la nourriture ou de l'eau à des doses de 100-200 millions de cellules par kg en poids 1 fois par 24 heures pendant 10 jours, ensuite à des doses équivalentes 2 fois par semaine. Pour le traitement, elle fut administrée à des doses de 100-200 millions de cellules par kg en poids par 24 heures pendant 10 jours. On n'utilise pas d'autres antibiotiques pour le traitement.

La préparation selon l'invention fut essayée dans un groupe de 3270 cochonnets à l'âge de 2 semaines à 2 mois. 164 animaux furent perdus (5,0%). Dans le groupe de témoins où la prophylaxie et le traitement étaient réalisés par les procédés connus auparavant, 74 animaux parmi 500 furent perdus (14,3%). L'augmentation journalière de poids dans le groupe d'essai fut 248 g, celle dans le groupe de témoins : 230 g.

Dans le groupe de 1781 cochons à l'âge de 2 à 4 mois, la préparation selon l'invention fut essayée. 26 cochons sont tombés (1,5%). Dans le groupe de témoins de la maladie d'oedème et de la dyspepsie, dans les mêmes délais, 140 (5,9%) animaux parmi 2387 sont tombés. Avec une ration identique de nourriture, l'augmentation de poids journalière fut de 346 g dans le groupe d'essai et de 283 g dans le groupe de témoins.

Certains cas de la maladie d'oedème et de la dyspepsie à la suite de l'utilisation prophylactique de la préparation selon l'invention, se déroulaient généralement sous une forme légère, sans perte de poids. On n'observera pas de mastites chez les truies durant toute la période d'essai, ni des complications.

La préparation selon l'invention fut introduite à raison de 0,5 à 2 g de poudre sèche dans la nourriture pour tous les animaux. Il n'y avait pas de complications avec l'utilisation de la préparation.

La préparation selon l'invention utilisée en pratique vétérinaire augmente notablement le poids des animaux. On peut donc l'utiliser dans le but prophylactique lors de l'élevage des jeunes animaux et pour l'amélioration de l'assimilation du fourrage.

La préparation selon l'invention est obtenue selon la technologie suivante.

On cultive la culture de bactéries sur l'agar à 3% de boeuf peptonisé, le lait ou sur le milieu de Blaurokk à une température de 36-37°C durant 24 heures. Ensuite, la culture est lavée par une solution à 0,9% de chlorure de sodium. La suspension obtenue est lyophilisée pendant 24 à 36 heures, à une pression de 40 à 80 Pa et à une différence de températures de -20 jusqu'à 40°C. On soumet la préparation obtenue aux examens visant à déterminer l'absence d'impuretés mécaniques, de contamination avec une microflore étrangère, la teneur en bactéries vivantes dans 1 mg, leur activité antagoniste, l'innocuité aux expériences avec les animaux suivant les méthodes généralement connues.

La série apte à l'utilisation est enregistrée et emballée.

Pour mieux comprendre l'essentiel de l'invention, on donne les exemples non limitatifs d'obtention de la préparation bactérienne suivants et de ses essais.

Exemple 1

On coule 200 matelas bactériologiques de 250 cm³ d'agar à 3% de boeuf peptonisé stérile. On introduit dans chaque matelas, en observant l'asepsie, 10 cm³ de suspension de souche Bacillus subtilis 534 contenant 100 millions de cellules dans 1 cm³ d'une solution à 0,9% de chlorure de sodium. Les matelas sont fermés avec des bouchons d'ouate-gaze et placés aux thermostats à une température de 36 à 37°C. Au bout de 24 heures, en observant l'asepsie, on lave la culture obtenue avec une solution à 0,9% de chlorure de sodium à raison de 100 cm³ pour 1 matelas bactériologique. On verse la suspension obtenue dans des flacons en verre de 500 cm³. on réalise la lyophilisation durant 24 heures à la pression de 70 Pa.

1 mg de préparation obtenue contient 250 millions de cellules vivantes de souche Bacillus subtilis 534. Les examens ont montré que la contamination avec une microflore étrangère et les impuretés mécaniques sont absentes dans la préparation obtenue.

La préparation inhibe la croissance du staphylocoque doré dans la zone de 30 mm, du protée dans la zone de 28 mm, des saccharomycètes dans la zone de 32 mm. L'introduction par voie interne de la préparation à 12 souris blanches a confirmé son innocuité. La série produite a été enregistrée et emballée. Son volume est de 10 milles doses pour l'homme.

Exemple 2.

La malade A., 59 ans. Diagnostic : aggravation de l'amygdalite chronique. Elle souffre depuis 48 heures, elle est intolérante aux antibiotiques, aux sulfanylamides. Le lait et la carotte provoquent la diarrhée et l'urticaire (éruptions sur la peau). On lui a administré la préparation à base de la culture vivante de la souche Bacillus subtilis 534 en dose de 5 milliards de cellules une fois par 24 heures. L'aggravation de l'amygdalite a été arrêtée à l'issue de 4 jours. On a prolongé le traitement à nouveau pendant 6 jours. On a inspecté la malade au bout de 6 mois. Elle est cliniquement saine. Elle mange en quantités modérées le lait et les carottes, ne marque pas d'allergie alimentaire.

Exemple 3

La malade M., 56 ans. Diagnostic : panaris osseum du deuxième doigt de la main gauche, lymphadénite. Elle est malade depuis deux mois. On a effectué deux fois l'abscision de l'abcès, et la séquestrectomie. Elle recevait des antibiotiques de large spectre d'action et des pansements avec les antibiotiques. On a proposé à la malade l'amputation du doigt. Le deuxième doigt est nettement oedémateux, cyanofique. Dans la région de la phalange moyenne, il y a une plaie de 1 cm de diamètre. Les granulations sont pâles, recouvertes de fibrine. La secrétion est séro-purulente. Les noeuds lymphatiques sont augmentés et douloureux. A la suite de l'examen bactériologique, le staphylocoque et les bactéries du genre Pseudomonas ont été isolées. On lui a administré par voie interne la préparation à base de la culture vivante de la souche Bacillus subtilis 534 à des doses de 5 milliards de cellules deux fois par 24 heures. Les pansements étaient avec une solution à 10% de chlorure de sodium. Au bout de 24 heures, la souche Bacillus subtilis 534 a été isolée dans la plaie. Au bout de 3 jours, la plaie s'est purifiée, à l'issue de 9 jours, la plaie a été épithélisée.

Exemple 4

La malade G., 48 ans. Diagnostic : septicémie chronique, infiltrat inflammatoire de l'avant-bras gauche, diabète sucré de gravité moyenne, dysbactériose.
Elle est malade depuis 1 ans et demi. A la suite des traumas microscopiques surgissaient des abcès, des furoncles et des phlegmons aux extrémités et au thorax. Elle a subi 9 opérations d'abcision et de drainage des abcès. Par la suite d'une longue utilisation des antibiotiques, s est développée la dysbactériose, elle était inquiétée par l'alternance des diarrhées et des constipations, le météorisme, elle surveillait la diète et recevait constamment l'insuline. Elle a été inspectée dans la clinique à la suite d'une aggravation et de la formation de l'infiltrat inflammatoire. Le staphylocoque doré a été isolé dans le sang. On a effectué le traitement durant 10 jours à partir de la préparation bactérienne à base de la culture vivante de la souche Bacillus subtilis 534 à des doses de 5 milliards de cellules 2 fois par 24 heures. La préparation lui a été administrée par voie interne. La malade ne recevait alors pas d'autres préparations antibactériennes. Après 48 heures, l'infiltrat inflammatoire a disparu. Dans l'ensemencement du sang, on n'a décelé que la souche Bacillus subtilis 534. Défécation normale.
Après 5 semaines, la malade s'est piquée à la main droite, il s'est formé l'infiltrat inflammatoire. On a réalisé le traitement du bras pendant 10 jours, a partir de la préparation selon l'invention à base de la culture vivante de la souche Bacillus sp. 538 qu'on lui a administrée par voie interne à des doses de 5 milliards de cellules 2 fois par 24 heures. L'infiltrat inflammatoire a disparu à l'issue de 5 jours.
La malade a été inspectée 2 ans après, elle observe la diète, reçoit de la maninile à la place de l'insuline. Les abcès n'apparaissent plus, la défécation est journalière, normale.

Exemple 5

Le malade K., 44 ans. Diagnostic : bronchopneumonie de gauche, malade depuis 6 jours. Plaintes de la hausse de la température à 38,5°C, de toux avec crachat, de faiblesse. Le diagnostic est confirmé par les données radiologiques. On a effectué le traitement à partir de la préparation selon l'invention à base de la culture vivante de la souche Bacillus sp. 538 à des doses de 2 milliards de cellules par 24 heures. On administrait la préparation per os pendant 10 jours. Le malade s'est guéri.

18

Exemple 6

La malade M., 53 ans. Diagnostic : pyélonéphrite bilatérale chronique, aggravation. Elle est malade depuis 9 ans. Les aggravations se répètent 5 à 8 fois par an, la dernière depuis 2 semaines. Le traitement par les préparations antibactériennes connues n'était pas efficace. Lors de l'inspection, elle avait des douleurs dans la région lombaire, des mictions fréquentes, une hausse de la température le soir. On a décelé par l'analyse microscopique dans l'urine, une grande quantité de leucocytes et des bactéries gram-négatives. Pour le traitement, on a utilisé la préparation contenant 15% de cellules de la souche Bacillus subtilis 534, 20% de cellules de Bacillus pulvifaciens 535, 65% de cellules Bacillus sp. 538. Elle lui était administrée à des doses de 0,5 milliard de cellules 2 fois pendant 15 jours. La température s'est normalisée à l'issue des 6 jours, les douleurs se sont apaisées. La pyurie a disparu vers le 9ème jour. On a isolé les bactéries du genre Bacillus dans le sang après 24 heures et dans l'urine 72 heures après l'administration de la première préparation.

Exemple 7

La malade K., 46 ans. Diagnostic : asthme bronchique, malade depuis 11 ans. Ce dernier mois, les accès d'étouffement étaient de 3 à 5 fois par 24 heures. Le traitement pathogénétique par les préparations connues ne donnait qu'un soulagement de courte durée. On a effectué le traitement par la préparation selon l'invention à base de la culture vivante de la souche Bacillus sp. 538. La préparation lui était administrée par voie interne à des doses de 3 milliards de cellules durant 10 jours. La quantité de crachat a augmenté à l'issue de 2 à 4 jours. La fréquence d'accès d'étouffement était peu à peu réduite. Elle était inspectée pendant 2 mois. Elle note l'amélioration de l'état. La respiration est plus libre. Les accès d'étouffement ne sont observés que 1 à 2 fois par semaine en présence d'odeurs fortes.

Exemple 8

Le malade V., 15 ans. Diagnostic : plaie purulente du pied droit. Il s'est coupé le pied avec du verre. Vers le 3ème jour apparut la secrétion purulente, la température du corps augmenta. On isola dans la plaie le staphylocoque doré. On effectua le traitement à partir de la préparation selon l'invention, à partir de la souche E. coli M17 administrée par voie interne à des doses de 1 milliard de cellules 2 fois durant 12 jours. On utilisait les pansements avec une solution à 10% de chlorure de sodium. La guérison s'est passée 11 jours après. La souche E. coli fut isolée dans le sang et la plaie, 48 heures après le traitement.

Exemple 9

Le malade P., à l'âge de 14 mois. Diagnostic : diathèse. Pendant 4 mois, à la peau de la figure et des mains, il y avait une éruption rouge vif. La démangeaison le tourmentait constamment. L'enfant reçoit la nutrition diététique. Le traitement par produits désensibilisants était inefficace. La préparation selon l'invention à base de la culture vivante de la souche Bacillius sp. 538 à des doses de 200 millions de cellules lui était administrée par voie interne avec la nourriture 2 fois durant 10 jours. L'éruption et la démangeaison ont disparu à l'issue de 7 jours. La souche Bacillus sp. 538 était isolée dans le sang et les matériaux de biopsie, 24 heures après la première introduction.

Exemple 10

La malade R., 3ans. Diagnostic : eczéma d'enfant. Elle est malade depuis deux ans et demi. L'affection s'était manifestée la première fois comme diathèse. Le traitement par les préparations désensibilisants, les enzymes protéolytiques, des onguents avec des corticostéroïdes et la diète était inefficace. Elle est cachectique. La peau du visage, du corps et des extrémités était toute recouverte d'écailles blanchâtres, alternant avec les vésicules. La peau porte des traces d'égratignures. On a réalisé le traitement (4 séries) par la préparation selon l'invention à base des souches Bacillus sp. 538 et Bacillus subtilis 534. Les préparations lui étaient administrées par voie interne à des doses de 1 milliard de cellules durant 10 jours. Les intervalles entre les séries étaient de 10 à 20 jours. La malade s'est guérie. On a isolé les bactéries du genre Bacillus dans le sang et les matériaux de biopsie de la peau, après 2 jours de traitement.

La malade a été inspectée au bout d'un an. La peau est douce, élastique, il n'y a pas d'éruptions. On l'avait dispensée de la diète. La malade augmenta son poids, son développement correspond à la norme physiologique.

Exemple 11

La préparation obtenue à base de la souche Bacillus subtilis 534 était essayée dans trois groupes de veaux à l'âge de 1 à 15 jours.

Le premier groupe (38 animaux) recevait par 5 mg (20 milliards de corps microbiens) de préparation, avec du lait 1 fois par jour durant 10 jours, ensuite à des doses équivalentes 2 fois par semaine. L'augmentation journalière du poids dans le groupe était de 715 g, 100% d'animaux étaient restés intacts.

Les animaux du deuxième groupe (37 animaux) recevaient la culture en bouillon propionique-acidophile à des doses journalières de 50-100 par animal une fois par jour. L'augmentation journalière du poids dans ce groupe était de 511 g. Deux animaux étaient perdus avec le diagnostic de la bronchopneumonie.

Le troisième groupe (31 animaux) était témoins. Durant deux mois, on a obtenu une augmentation journalière du poids de 391 g, un veau est perdu avec le diagnostic de la bronchopneumonie.

Exemple 12

La préparation selon l'invention à base de la souche Bacillus pulvifaciens 535 à des doses de 50 à 100 milliards de cellules, 2 fois par 24 heures, avec le fourrage a été administrée à 16 vaches souffrant de l'endométrite. 13 animaux s'étaient guéris à l'issue de 7 à 8 jours sans traitement complémentaire. 4174 vaches avant le vêlage recevaient cette préparation à des doses de 10 à 50 milliards de cellules avec le fourrage 1 fois par jour : l'endométrite s'était développée chez 17% d'animaux, tandis que chez 5065 vaches du groupe témoins : dans 80% des cas.

Exemple 13

Pour les essais de la préparation bactérienne selon l'invention obtenue à partir de la souche Bacillus pulvifaciens 535, on a pris deux groupes de poussins de la race pondeuse, à l'âge d'un jour.

Le premier groupe (1368 poussins) recevait la préparation à des doses de 500 millions de corps microbiens par kg de la masse vivante avec la nourriture 1 fois par jour durant 10 jours, ensuite à des mêmes doses 1 fois tous les trois jours.

Le deuxième groupe (1420) poussins était le témoin.

Il fut établi qu'à l'âge de 10 jours, l'augmentation du poids moyen dans le groupe d'essai était de 11,5% et à l'âge de 20 jours de 10,2% supérieure à celle du groupe témoin.

La perte des poussins durant 20 jours d'élevage constitua 6,36% dans le groupe d'essai et 7,32 dans le groupe témoin.

La préparation bactérienne selon l'invention trouve application en médecine et en médecine vétérinaire. La préparation selon l'invention peut être utilisée pour la prophylaxie et le traitement des processus pathologiques suivants :

a) affections inflammatoires provoquées par les bactéries gram-positives et gram-négatives, les mycètes (plaies purulentes, abcès, phlegmons, lymphangite furoncles, anthrax, septicémie, pneumonies, péritonites, cholécystite, endométrite, pyélonéphrite, diarrhée, etc.) ;

b) affections allergiques (diathèses, eczéma, asthme bronchique, intolérances des préparations médicinales et des produits alimentaires) ;

c) troubles de la digestion provoqués par le déficit en enzymes protéolytiques propres ;

d) dysbactérioses.

En outre, la préparation peut être utilisée en médecine vétérinaire pour l'amélioration de l'assimilation du fourrage. Elle permet de réduire le volume de ration indispensable à la nutrition.

**Revendications**

1. Préparation bactérienne pour la prophylaxie et le traitement des processus inflammatoires et des maladies allergiques, caractérisée en ce qu'elle représente des cellules d'au moins un micro-organisme de bactéries choisi parmi les souches Bacillus subtilis 534, Bacillus sp 538 et Bacillus pulvifaciens 535, apte à pénétrer du système digestif dans le foyer d'affection, à conserver sa viabilité, à produire des antibiotiques, des ferments protéolytiques, des modulateurs d'immunité, et en ce qu'elle contient de 5 à $1.10^9$ de cellules vivantes de micro-organismes par mg de préparation.

2. Préparation bactérienne selon la revendication 1, caractérisée en ce que la souche Bacillus subtilis 534 a été déposée le 28.03.1988 dans la Collection d'URSS des micro-organismes de l'Institut biokhimii i fiziologii mikroorganiznov de l'Académie des Sciences de l'URSS et enregistrée sous le numéro B-1666 II.

**3.** Préparation bactérienne selon la revendication 1, caractérisée en ce que la souche Bacillus Sp. 538 a été déposée le 20.06.1988 à la Collection d'URSS des micro-organismes du Vsesojuzny nauchno-issledovatelsky institut genetiki i selektsii promyshlennyh mikroorganiznov et enregistrée sous le numéro BKII M B-4401.

**4.** Préparation bactérienne selon la revendication 1, caractérisée en ce que la souche Bacillus pulvifaciens 535 a été déposée le 14.04.1988 dans la collection nationale des micro-organismes industriels du Vsesojuzny nauchno-issledovatelsky institut genetiki i selektsii promyshlennykh mikroorganizmov et enregistrée sous le numéro BKIIM B-4348.

**5.** Utilisation de la souche Escherichia Coli M17 pour l'obtention d'une préparation bactérienne selon la revendication 1 destinée à traiter des processus purulents dans les tissus musculaires des extrémités.

**Claims**

**1.** Bacterial preparation for the prophylaxis and the treatment of inflammatory processes and allergic diseases, characterized in that it exhibits cells of at least one micro-organism of bacteria selected among the Bacillus subtilis 534, Bacillus sp 538 and Bacillus pulvifaciens 535 strains, apt to penetrate from the digestive system into the center of disease, to preserve its viability, to produce antibiotics, proteolytic ferments, immunity modulators and in that it contains from 5 to $1.10^9$ living cells of micro-organisms per mg of preparation.

**2.** Bacterial preparation according to claim 1, characterized in that the Bacillus subtilis 534 strain has been deposited on March 28, 1988 in the Collection of the U.S.S.R. of the micro-organisms of the Institut biokhimii i fiziologii mikroorganiznov of the Academy of Sciences of the U.S.S.R. and registered under the number B-1666 II.

**3.** Bacterial preparation according to claim 1, characterized in that the Bacillus sp. 538 strain has been deposited on June 20, 1988 in the Collection of the U.S.S.R. of the micro-organisms of the Vsesojuzny nauchno-issledovatelsky institut genetiki i selektsii promyshlennyh mikroorganiznov and registered under the number BKII M B-4401.

**4.** Bacterial preparation according to claim 1, characterized in that the Bacillus pulvifaciens 535 strain has been deposited on April 14, 1988 in the national collection of the industrial micro-organisms of the Vsesojuzny nauchno-issledovatelsky institut genetiki i selektsii promyshlennykh mikroorganizmov and registered under the number BKII M B-4348.

**5.** Utilization of the Escherichia Coli M17 strain for the obtaining of a bacterial preparation according to claim 1, intended to treat purulent processes in the muscular tissues of the limbs.

**Patentansprüche**

**1.** Bakterielle Zubereitung für die Prophylaxe und die Behandlung von Entzündungsvorgängen und von allergischen Krankheiten, dadurch gekennzeichnet, dass sie Zellen von wenigstens einem unter den Bacillus subtilis 534, Bacillus sp 538 und Bacillus pulvifaciens 535 Stämmen gewählten Mikroorganismus von Bakterien darstellt, welcher fähig ist, von dem Verdauungssystem in den Krankheitsherd einzudringen, seine Lebensfähigkeit zu bewahren, Antibiotiken, proteolytische Gärmittel, Immunitätsmodulatore zu erzeugen und dass sie von 5 bis $1.10^9$ lebende Zellen von Mikroorganismen je mg der Zubereitung enthält.

**2.** Bakterielle Zubereitung gemäss Anspruch 1, dadurch gekennzeichnet, dass der Bacillus subtilis 534-Stamm am 28. März 1988 in der Sammlung der U.S.S.R. von Mikroorganismen des Institut biokhimii i fiziologii mikroorganiznov der Akademie der Wissenschaften der U.S.S.R. hinterlegt und unter der Nummer B-1666 II eingetragen worden ist.

**3.** Bakterielle Zubereitung nach Anspruch 1, dadurch gekennzeichnet, dass der Bacillus sp. 538-Stamm am 20. Juni 1988 in der Sammlung der U.S.S.R. der Mikroorganismen des Vsesojuzny nauchno-issledovatelsky institut genetiki i selektsii promyshlennyh mikroorganiznov hinterlegt und unter der Nummer BKII M B-4401 eingetragen worden ist.

**4.** Bakterielle Zubereitung nach Anspruch 1, dadurch gekennzeichnet, dass der Bacillus pulvifaciens 535-Stamm am 14. April 1988 in der nationalen Sammlung der gewerblichen Mikroorganismen des Vsesojuzny nauchno-issledovatelsky institut genetiki i selektsii promyshlennykh mikroorganizmov hinterlegt und unter der Nummer BKIIM B-4348 eingetragen worden ist.

**5.** Verwendung des Escherichia Coli M17 Stammes zur Erhaltung einer bakteriellen Zubereitung gemäss dem Anspruch 1, die bestimmt ist, vereiterte Vorgänge in den Muskelgeweben der Glieder zu behandeln.